# FASCICULE DE BREVET EUROPEEN

(11) **EP 0 688 749 B1**
(45) Date de publication et mention de la délivrance du brevet: **07.10.1998**
(21) Numéro de dépôt: 95401355.3
(22) Date de dépôt: 12.06.1995
(51) Int. Cl.: C07C 2/58, B01J 38/56

(54) **Procédé de réjuvénation d'un catalyseur solide d'alkylation**
Verfahren zur Verjüngung eines festen Alkylierungskatalysators
Process for rejuvenating a solid alkylation catalyst

(30) Priorité: 22.06.1994 FR 9407855
(43) Date de publication de la demande: 27.12.1995
(73) Titulaire: INSTITUT FRANCAIS DU PETROLE, 92502 Rueil-Malmaison (FR)
(72) Inventeur: Joly, Jean-François, F-69006 Lyon (FR); Benazzi, Eric, F-78360 Montesson (FR); Marcilly, Christian, F-78800 Houilles (FR); Euzen, Jean-Paul, F-69570 Dardilly (FR); Forestiere, Alain, F-69390 Vernaison (FR)

(56) Documents cités:
- EP-A- 0 584 006
- US-A- 3 729 526
- US-A- 4 071 576

## Description

La présente invention concerne un procédé de réjuvénation d'un catalyseur solide d'alkylation d'au moins une isoparaffine, de préférence l'isobutane, par au moins une oléfine contenant de 3 à 6 atomes de carbone par molécule.

On sait que, pour alimenter les moteurs à combustion interne et à allumage commandé, et notamment les moteurs à taux de compression élevé, il est particulièrement intéressant de disposer de carburants à hauts indices d'octane, c'est-à-dire constitués essentiellement par des hydrocarbures paraffiniques fortement ramifiés. L'alkylation des isoparaffines (isobutane et/ou isopentane) par les oléfines contenant de 3 à 6 atomes de carbone par molécule permet d'obtenir de tels produits. Cette réaction nécessite la mise en oeuvre de catalyseurs très acides, dans le but notamment de réduire les réactions parasites telles que les réactions d'abstraction d'hydrure de l'oléfine et de polymérisation qui fournissent des hydrocarbures peu ramifiés de faible indice d'octane et des hydrocarbures insaturés, les réactions de craquage et les réactions de dismutation.

Les procédés existant pour la production d'hydrocarbures par alkylation d'isoparaffine par les oléfines utilisent soit l'acide sulfurique soit l'acide fluorhydrique comme catalyseur. Dans ces procédés le catalyseur acide constitue une phase liquide qui est mise en contact avec le mélange isobutane-oléfine liquide pour former une émulsion. Ces procédés sont coûteux et posent d'importants problèmes vis-à-vis de la sécurité des personnes et de l'environnement. Afin de remédier à ces problèmes, des systèmes catalytiques différents des acides sulfurique et fluorhydrique en phase liquide ont été recherchés.

La présente invention concerne un procédé d'alkylation catalytique d'isoparaffines (notamment l'isobutane et/ou l'isopentane) au moyen d'une oléfine, qui permet d'obtenir au moins un produit par exemple appartenant au groupe constitué par les diméthylbutanes, les triméthylpentanes, les triméthylhexanes et les triméthylheptanes, procédé dans lequel l'oléfine et/ou un mélange d'oléfines est introduit(e) dans la zone de réaction en phase liquide et en mélange avec l'isoparaffine et/ou un mélange d'isoparaffines.

Le procédé selon la présente invention permet de réaliser dans les meilleures conditions la réaction d'alkylation de l'isobutane par les oléfines, réaction qui est caractérisée par une forte exothermicité (environ 20 kcal/Mole d'oléfine (butène notamment) de butène transformé). Le procédé permet en particulier d'obtenir une bonne homogénéité de température et de concentration en réactifs

Dans le procédé d'alkylation d'au moins une isoparaffine selon la présente invention, les conditions opératoires, et plus particulièrement la température et la pression, sont choisies de façon à ce que le mélange constitué par l'isobutane, les oléfines et les produits de la récation soit liquide. De plus, il est important que le catalyseur baigne dans ledit liquide de façon à assurer partout un bon contact liquide-solide. Ce faisant on évite l'apparition de zones sèches dans le réacteur, lesquelles zones pourraient être responsables d'un manque de stabilité thermique, ces zones sèches pouvant atteindre des températures élevées du fait de la réaction qui peut se dérouler entièrement en phase gazeuse à ces endroits. Aussi a-t-on proposé diverses techniques, utilisant une phase liquide continue, le catalyseur pouvant être utilisé sous forme de suspension, sous forme de lit bouillonnant ou sous forme de lit fixe ou mobile.

Parmi les procédés qui utilisent des catalyseurs acides solides on peut citer les solides acides suivants : les tamis moléculaires, les résines macroréticulaires éventuellement associées avec le trifluorure de bore (BF₃), les résines perfluorées, les acides de Lewis et/ou de Brönsted déposés sur différents supports inorganiques, des alumines chlorées, les graphites intercalés par des acides de Lewis et/ou de Brönsted et les anions déposés sur des supports oxydes tels que ZrO₂/SO₄. Parmi ces procédés on peut en distinguer deux types : (i) le catalyseur est mis en suspension dans une phase hydrocarbonée par agitation vigoureuse au sein du réacteur : c'est le cas du réacteur parfaitement agité ; (ii) le catalyseur est en lit fixe dans le réacteur.

Parmi les procédés de type (i), on peut citer les brevets US-A-3879489 et US-A-3855343 ; ce type de mise en oeuvre présente un inconvénient majeur : le catalyseur peut être rapidement détruit par attrition, ce qui pose le problème de l'élimination des fines. Une amélioration de la mise en oeuvre de procédé de type (ii) décrite dans le brevet US-A-3852371, qui propose une injection de la charge isoparaffine(s)-oléfine(s) à différentes hauteurs dans le lit de catalyseur, avec recyclage partiel à ces mêmes hauteurs, ce qui paraît plus complexe à maîtriser. D'autre part, les brevets US-A-3852371 et US-A-5073665, qui font état d'une mise en oeuvre préférée en lit fixe, décrivent le recyclage partiel de l'effluent sortant du réacteur en entrée du réacteur, le brevet US-A-5073665 fait état, dans le cas d'une mise en oeuvre comportant au moins un lit fixe de catalyseur, d'une alimentation en oléfine(s) pour chaque lit fixe de catalyseur.

Le brevet US-A-5157196 revendique l'utilisation de catalyseurs d'alkylation en lit circulant, ladite invention est en particulier caractérisée en ce qu'une zone de lavage, différente de la zone de réaction, du catalyseur est présente et fonctionne en lit fluide, de plus les temps de séjour des hydrocarbures dans le réacteur est court (1 seconde à 5 minutes).

Le brevet EP-A-584.006 décrit un procédé en continu avec le recyclage partiel de l'effluent soutiré de la zone réactionnelle vers ladite zone réactionnelle et le recyclage vers la zone réactionnelle d'une fraction riche en isoparaffine recueillie après séparation de l'effluent réactionnel.

La présente invention concerne un procédé d'alkylation d'isoparaffines (isobutane et/ou isopentane), en présence d'un catalyseur hétérogène, et caractérisé en ce que le catalyseur est dans une zone réactionnelle R en suspension dans une phase d'hydrocarbures à l'état liquide. La zone R peut fonctionner en lit fixe, en lit expansé, en lit mobile ou bien encore en réacteur parfaitement agité (Grignard), bouillonnant, fluide ou toute autre forme équivalente.

De façon plus précise, l'invention concerne donc un procédé de réjuvénation d'un catalyseur solide d'alkylation d'au moins une isoparaffine par au moins une oléfine comportant de 3 à 6 atomes de carbone par molécule caractérisé en ce que ladite réjuvénation est effectuée dans la zone de réaction au moins périodiquement, par circulation sur le lit catalytique (donc par lavage de ce catalyseur) d'au moins une fraction riche en isobutane obtenue après fractionnement ou séparation de l'effluent réactionnel. Les moyens mis en oeuvre dans la présente invention pour effectuer le lavage du catalyseur (que celui ci soit dans la zone réactionnelle sous la forme d'un lit fixe expansé, bouillonnant, fluide, agité ou toute forme équivalente) sont donc essentiellement des recyclages de fractions de l'effluent réactionnel spécifiquement choisies. Le ou les recyclages en question sont effectués au moins en partie à l'entrée de la zone réactionnelle (c'est-à-dire généralement à la base inférieure de la zone réactionnelle) mais également de façon éventuellement préférée à divers niveaux de la zone réactionnelle pour que l'intégralité du lit catalytique soit au contact des effluents de recyclage.

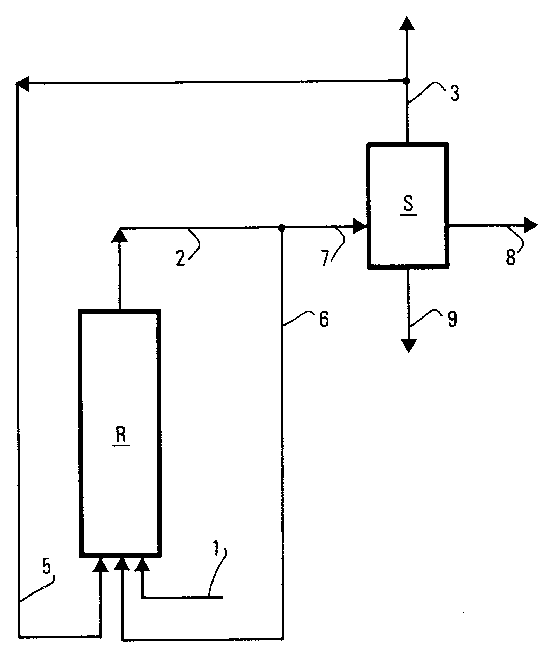

Le procédé selon l'invention est caractérisé également en ce que l'on opère périodiquement un traitement dit de réjuvénation qui comprend à titre d'exemple au moins les quatre étapes suivantes illustrée sur la figure unique :
- étape 1 : l'alimentation d'une zone réactionnelle R renfermant un lit catalytique par une charge (i) introduite par une conduite 1 (la charge étant un mélange d'oléfines et d'isoparaffine) est stoppée.
- étape 2 : le catalyseur est généralement séparé de la phase hydrocarbure liquide présente dans la zone réactionnelle R. La phase liquide est alors soutirée par une conduite 2 de la zone réactionnelle R pour être envoyée vers une zone de séparation S. Le catalyseur reste présent dans la zone réactionnelle R, pendant ledit soutirage.
- étape 3 : une phase liquide (conduite 3) riche en isoparaffine, provenant de la tête de la zone de séparation S (composé (iii)), est introduite au moins en partie dans la zone réactionnelle R par la ligne 5, le catalyseur à ce stade peut être mis en suspension dans ladite phase liquide.

Cette opération de rinçage peut être répétée à plusieurs reprises et l'effluent de lavage peut être à chaque fois renvoyé dans la zone normale de séparation S.

Les étapes 2 et 3 peuvent être faites en une seule étape ; lorsque l'on décide de pratiquer la réjuvénation, le réacteur concerné est directement relié à la section de séparation S et n'est plus alimenté par les lignes 3 et 5 que par l'effluent léger de la section S (principalement de l'isoparaffine).

Le lavage peut évidemment être effectué en co-courant ou en contre-courant, dans le cas où le catalyseur est disposé en lit expansé.
- étape 4 : les conditions de température et de pression sont amenées aux valeurs correspondant à celles de la réaction d'alkylation, on introduit dans la zone réactionnelle R, la charge (i) décrite dans l'étape 1 ci-dessus qui arrive donc au contact par exemple d'un catalyseur noyé dans l'isoparaffine si l'on opère en lit fixe ou d'une suspension de catalyseur dans l'isoparaffine si l'on opère en lit expansé, agité de type Grignard.

Les étapes 2 et 3 peuvent être répétées plusieurs fois.

Le traitement de réjuvénation du catalyseur selon la présente invention, décrit par la succession des étapes décrites ci-dessus, est repété périodiquement au cours du temps. Ce traitement n'est pas une régénération du catalyseur mais un traitement qui permet de conserver suffisamment d'activité au catalyseur pour qu'il ne soit pas nécessaire de le régénérer avant un long intervalle de temps. Le catalyseur d'alkylation est donc soumis à des cycles de réaction-réjuvénation. L'un des avantages du procédés selon la présente invention est que le catalyseur reste en permanence présent au sein de la zone réactionnelle R, lors desdits cycles réaction-réjuvénation.

Le procédé de la présente invention s'applique à tout catalyseur hétérogène permettant de réaliser l'alkylation d'isoparaffine, et plus particulièrement de l'isobutane et/ou de l'isopentane, par des oléfines comprenant de 3 à 6 atomes de carbone par molécule.

Le catalyseur présent dans la zone R est choisi parmi les catalyseurs solides connus de l'homme du métier. De préférence, le catalyseur est choisi parmi les catalyseurs suivants :
* un catalyseur comprenant au moins de l'acide sulfurique imprégné sur un support poreux organique ou minéral, tels les catalyseurs décrits dans les demandes de brevet français 2682891, 2683740, 2683739 et 2687935.
* un catalyseur comprenant le mélange contenant d'une part au moins un halogénure de composé choisi dans le groupe formé par l'aluminium et le bore et d'autre part au moins un halogénure d'ammonium quartenaire et/ou halohydrate d'amine, tel par exemple que le catalyseur décrit dans la demande de brevet français 2686526.

Le catalyseur utilisé dans la présente invention, renferme de préférence, de la silice et de l'acide sulfurique, la silice étant imprégnée totalement par l'acide sulfurique. La silice est caractérisée en ce que son volume poreux total soit supérieur à 0,5 cm³/g. Le catalyseur obtenu après imprégnation est caractérisé en ce que la teneur pondérale en acide sulfurique est supérieure à 40%, et de préférence supérieur à 70%.

La silice peut contenir des impuretés comme par exemple les oxydes, les alcalins, les alcalinoterreux, les composés d'aluminium ou toute autre impureté connue de l'homme du métier, la quantité totale de ces impuretés n'excédant pas 2 % en poids par rapport à la silice.

La concentration de l'acide sulfurique est avantageusement comprise entre 90 et 100 % en poids, de préférence entre 97 et 100 % en poids et de manière encore plus préférée entre 98 et 100 % en poids

Il est possible d'ajouter dans la phase acide H₂SO₄, avant l'imprégnation, des additifs visant à améliorer les performances catalytiques. Parmi ces additifs on peut citer à titre d'exemple l'acide trifluorométhanesulfonique CF₃SO₃H, et l'acide HB(SO₄H)₄. Les catalyseurs préférés utilisés pour la présente invention (du type acide sulfurique sur silice, dopé de préférence par un composé du bore) donnent des résultats supérieurs à ceux que l'on obtiendrait avec des catalyseurs plus conventionnels du type silice dopée par SbF₃ ou SbF₅.

Le diamètre moyen des particules du catalyseur, principalement constitué de grains sensiblement sphériques, utilisé dans le procédé selon la présente invention est généralement compris entre 0.1 et 150 microns (1 micron = 10⁻⁶ m), de préférence entre 5 à 110 microns et d'une façon encore plus préférée entre 5 et 80 microns.

Le procédé de la présente invention est un procédé d'alkylation dans lequel on traite une charge comprenant d'une part au moins une isoparaffine, laquelle de préférence est choisie dans le groupe formé par l'isobutane et l'isopentane, de manière encore plus préférée est l'isobutane, et d'autre part au moins une oléfine contenant de 3 à 6 atomes de carbone par molécule, en présence d'un catalyseur solide d'alkylation ; ledit procédé comprend :
a) l'introduction et la mise en contact avec le catalyseur présent dans une zone réactionnelle R des composés ou éléments suivants :
   (i) ladite charge, de préférence introduite au moins à l'entrée de la zone R, par la conduite 1,
   (ii) éventuellement l'effluent liquide décrit en b), introduit au moins à l'entrée de la zone R, de préférence introduit totalement à l'entrée de la zone R, et,
   (iii) l'effluent liquide décrit en d), de préférence introduit au moins à l'entrée de la zone R ,
b) éventuellement le recyclage par la conduite 6 d'une partie de l'effluent réactionnel liquide sortant par la conduite 2 de la zone réactionnelle R vers l'entrée de ladite zone R,
c) l'introduction par la conduite 7 d'au moins une partie de l'effluent liquide sortant de la zone réactionnelle R dans une zone S de fractionnement ou de séparation isobutane/normal-paraffine/alkylat,
d) le recyclage de la majeure partie de l'effluent liquide riche en isoparaffine soutiré par les conduites 3 et 5 de la zone S en zone réactionnelle R,
e) l'obtention d'un alkylat à titre de produit, soutiré par la conduite 8 de la partie inférieure dans la zone S, et,
f) l'obtention conduite 9 de normal-paraffine à titre de purge de la zone S.

Le procédé selon la présente invention est également caractérisé en ce que le catalyseur est périodiquement réjuvéné selon le procédé en cinq étapes décrit ci-avant, avec pendant la réjuvénation proprement dite, arrêt de l'introduction de la charge (i) dans la zone réactionnelle (R).

Selon une mise en oeuvre préférée du procédé selon l'invention, il est possible d'introduire la charge, c'est-à-dire le composé (i) décrit en a), en plusieurs points de la zone R.

Selon une autre mise en oeuvre préférée du procédé selon l'invention, il est possible d'introduire le composé (ii) décrit en a), en plusieurs points de la zone R et plus particulièrement à différents niveaux du lit catalytique.

De préférence, les fractions des composés (i) à (iii) décrits en a) qui sont introduites dans la zone R sont mélangées ensemble en partie ou en totalité, de préférence en totalité, avant d'être introduites dans ladite zone.

La température dans la zone réactionnelle R est généralement comprise entre -15 et +6°C, de préférence entre -15 et 0°C.

De préférence, la charge a été séchée sur tamis moléculaire et hydrogénée sélectivement avant son introduction dans la zone réactionnelle R de manière à éliminer la majeure partie au moins des composés très fortement insaturés que la charge peut éventuellemnt contenir susceptibles d'inhiber la phase catalytique.

Les réactifs sont introduits de façon à ce que la vitesse spatiale horaire, exprimée en poids d'oléfine(s) introduite(s) par unité de poids du catalyseur présent dans la zone R et par heure, soit généralement comprise entre 0,01 et 10 h⁻¹, de préférence entre 0,02 et 2 h⁻¹, et de manière encore plus préférée entre 0,05 et 1 h⁻¹.

La concentration volumique de catalyseur dans la zone réactionnelle R, exprimée en volume de catalyseur par volume de phase liquide hydrocarbure est comprise entre 1:100 et 1:1 et de préférence entre 1:100 et 1:50, et d'une façon encore plus préférée entre 1:50 et 1:4.

Afin de limiter les réactions secondaires, on utilise un excès d'isoparaffine par rapport à l'oléfine à l'entrée de la zone réactionnelle R. A titre d'exemple, dans le cas de l'alkylation de l'isobutane par un butène, l'isobutane peut être introduit pur dans la charge ou sous la forme d'un mélange de butanes contenant par exemple au moins 40 % d'isobutane. De plus, on peut introduire un butène pur ou encore un mélange de butènes isomères. Dans tous les cas, le rapport molaire isobutane/butènes à l'entrée de la zone réactionnelle R est généralement compris entre 5 et 1000, de préférence entre 5 et 500 et de manière souvent préférée entre 10 et 200.

De façon à limiter les réactions secondaires de dégradation des isoparaffines C₅-C₁₂ présentes dans les effluents liquides passant au travers de(s) zone(s) de réactions R, on réalise l'opération de séparation ou de fractionnemnt dans la zone S de façon à ce que le rapport des débits molaires d'isoparaffine (par exemple d'isobutane) en tête de la zone de séparation S et d'alkylat en fond de la zone S soit compris entre 5:1 et 100:1, et de préférence compris entre 10:1 et 50:1.

Le traitement de réjuvénation du lit de catalyseur utilisé dans la présente invention et présent dans la zone réactionnelle R est effectué à une périodicité (ou cycle) correspondant à des ages du catalyseur, exprimé en poids d'alkylat produit par poids de catalyseur, compris entre 0,01 et 50 g/g, de préférence entre 0,1 et 25 g/g et d'une manière encore plus préférée entre 0,1 et 5 g/g. La durée correspondant à la réalisation des 4 étapes décrites ci-avant est avantageusement comprise entre 1 minute et 10 heures, et d'une manière souvent préférée entre 1 minute et 5 heures.

Un ou plusieurs réacteurs peuvent être utilisés dans le procédé selon l'invention, dans le cas ou l'on utilise plusieurs réacteurs en série, il est avantageux d'introduire l'isoparaffine de la charge (composé (i)) et provenant de la zone de séparation S (composé (iii)) à l'entrée du premier réacteur, les oléfines sont alors introduites à l'entrée de chacun des réacteurs.

Si l'on utilise des réacteurs en parallèle, chacun des réacteurs recevra une partie de l'effluent commun (recyle) ainsi que les quantités appropriées d'oléfines (par exemple des butènes) (frais et recyclé depuis la tête de la colonne à distiller S) et ceci au travers de systèmes de répartition bien connus de l'homme de l'art.

Dans le cas ou l'on utilise plusieurs réacteurs, au mois un réacteur se trouve en cycle de réjuvénation, les autres étant en cycle de réaction (technique dite "swing").

Dans une réalisation préférée du procédé selon la présente invention on peut renouveler le contenu en catalyseur des réacteurs. Pour cela on soutire au moins une partie du catalyseur désactivé, c'est-à-dire à l'issue de plusieurs traitements de réjuvénation, maintenu en suspension par tout moyen connu de l'homme de l'art et on introduit du catalyseur frais ou du catalyseur régénéré ex situ dans la zone de réaction R. Les quantités de catalyseur soutirées sont égales aux quantités de catalyseurs introduites. Cette opération de remplacement interviendra de préférence à la fin d'une opération de réjuvénation.

## Revendications

1. Procédé de réjuvénation d'un catalyseur solide d'alkylation d'au moins une isoparaffine par au moins une oléfine comportant de 3 à 6 atomes de carbone par molécule, la dite réjuvénation étant effectuée dans la zone de réaction, par circulation sur le lit catalytique d'au moins une partie d'une fraction riche en isoparaffine obtenue par fractionnement ou séparation de I'effluent réactionnel liquide en provenance de la zone réactionnelle, le procédé comprenant l'alimentation d'une zone réactionnelle R renfermant un lit catalytique par une charge (i) renfermant au moins la dite isoparaffine et la dite oléfine introduite au moins en partie à la base de la zone réactionnelle, caractérisé en ce que :
- périodiquement au moins l'alimentation est arrétée,
- on procède au soutirage de l'effluent réactionnel, le lit catalytique restant substantiellement dans la zone réactionnelle (R),
- on sépare ou on fractionne dans une zone S l'effluent réactionnel en vue de recueillir au moins une fraction riche en isoparaffine, une partie au moins de cette fraction étant recyclée dans la zone réactionnelle afin de laver le dit catalyseur
- on procède au redémarrage de la réaction en réalimentant la zone réactionnelle par la charge (i) avec, si nécessaire, ajustement des conditions opératoires nécessaires à la réaction d'alkylation.

2. Procédé selon la revendication 1 dans lequel l'isoparaffine est choisie dans le groupe constitué par l'isobutane et l'isopentane.

3. Procédé selon l'une des revendications 1 et 2 dans lequel le lit catalytique est un lit choisi dans le groupe constitué par les lits fixe, expansé, bouillonnant, agité ou de nature équivalente.

4. Procédé selon l'une des revendications 1 à 3 dans lequel le soutirage de l'effluent réactionnel hors de la zone réactionnelle et le recyclage dans la dite zone réactionnelle d'une fraction riche en isoparaffine sont effectués sensiblement simultanément.

5. Procédé selon l'une des revendications 4 et 5 caractérisé en ce qu'il est répété périodiquement.

6. Procédé selon l'une des revendications 1 à 5 dans lequel la charge (i) est introduite en plusieurs points de la zone réactionnelle, ces points étant situés à différentes hauteurs.

7. Procédé selon l'une des revendications 4 à 6 dans lequel :
(a) on introduit dans la zone réactionnelle les composés ou éléments suivants :
(i) une charge,
(ii) éventuellement une partie au moins de l'effluent réactionnel,
(iii) une fraction riche en isoparaffine provenant de l'effluent réactionnel,
(b) on soutire, après arrêt éventuel de la réaction d'alkylation, l'effluent réactionnel et l'on recycle éventuellement vers la zone réactionnelle une partie de cet effluent réactionnel désignée (ii) à l'étape (a),
(c) on envoie une partie au moins du dit effluent réactionnel dans une zone S de séparation ou de fractionnement,
(d) on recycle vers la zone réactionnelle une partie au moins d'une fraction riche en isoparaffine soutirée de la dite zone S et désignée (iii) l'étape (a),
(e) on récupère à partir de la zone S un alkylat à titre de produit.

8. Procédé selon la revendication 7 dans lequel en outre on récupère à titre de purge une n-paraffine dans la zone S.

9. Procédé selon l'une des revendications 7 et 8 dans lequel le lit catalytique est lavé dans la zone réactionnelle au moyen de la dite fraction riche en isoparaffine soutirée de la dite zone S et désignée (iii) à l'étape (a).

10. Procédé selon l'une des revendications 7 à 9 dans lequel le recyclage réalisé en (a) d'une partie de l'effluent réactionnel (ii) est effectué à plusieurs niveaux de la zone réactionnelle.

11. Procédé selon l'une des revendications 7 à 10 dans lequel le recyclage réalisé en (a) d'une partie de la fraction riche en isoparaffine (iii) est effectué à différents niveaux du lit catalytique.

12. Procédé selon l'une des revendications 7 à 11 dans lequel à l'étape (a), les éléments (i), (ii) et éventuelement (iii) sont mélangés avant d'être introduits dans la zone réactionnelle R.

13. Procédé selon l'une des revendications 1 à 12 dans lequel au préalable, avant l'introduction de la charge dans la zone réactionnelle, cette charge a été séchée sur un tamis moléculaire et hydrogénée sélectivement de manière à éliminer la majeure partie au moins des composés fortement insaturés qu'elle peut contenir.

14. Procédé selon l'une des revendications 4 à 13 dans lequel le rapport des débits molaires d'isoparaffine soutiré de la zone de séparation ou de fractionnement S et de l'alkylat soutiré de cette même zone soit compris entre 5:1 et 100:1.

15. Procédé selon la revendication 14 dans lequel le dit rapport est compris entre 10:1 et 50:1.

16. Procédé selon l'une des revendications 1 à 15 dans lequel un traitement de réjuvénation d'un lit catalytique est effectué avec une périodicité correspondant à un age du catalyseur (exprimé en poids d'alkylat produit par poids de catalyseur) compris entre 0,01 et 50 g/g.

17. Procédé selon la revendication 4 caractérisé en ce que la durée de ce procédé est comprises entre 1 minute et 10 heures.

18. Procédé selon l'une des revendications 1 à 17 dans lequel la réaction d'alkylation est effectuée dans au moins deux zones de réaction disposées en série et dans lequel l'isoparaffine à traiter est introduite dans la première zone de réaction traversée par la charge, dans lequel l'oléfine est introduite dans chaque zone de réaction et dans lequel l'isoparaffine soutirée de la zone S est recyclée vers la première zone de réaction.

19. Procédé selon l'une des revendications 1 à 17 dans lequel la réaction d'alkylation est effectuée dans au moins deux zones de réaction disposées en parallèle, chaque zone de réaction étant alimentée en oléfine à traiter en isoparaffine à traiter et en isoparaffine de recyclage en provenance de la zone S.

20. Procédé selon l'une des revendications 1 à 19 dans lequel, à l'issue de plusieurs traitements de réjuvénation catalytique, on soutire au moins une partie du lit catalytique, la dite partie du lit catalytique étant remplacée par du catalyseur régénéré ou du catalyseur frais.

## Claims

1. A process for the rejuvenation of a solid catalyst for the alkylation of at least one isoparaffin by at least one olefin containing 3 to 6 carbon atoms per molecule, said rejuvenation being carried out in the reaction zone, at least periodically, by circulating at least a portion of an isoparaffin-rich fraction obtained by fractionation or separation of the liquid reaction effluent from the reaction zone over the catalytic bed, the process being characterised in that the reaction zone R containing a catalytic bed is supplied with a feed (i) comprising at least said isoparaffin and said olefin introduced at least partially to the base of the reaction zone,
characterised in that :
- at least the supply is periodically stopped,
- the reaction effluent is extracted and the catalytic bed remains substantially in the reaction zone R,
- the reaction effluent is separated or fractionated in a zone S to recover at least an isoparaffin-rich fraction, at least a portion of this fraction being recycled to the reaction zone to wash said catalyst,
- the reaction is restarted by restarting the supply of feed (i) to the reaction zone, adjusting the operating conditions required for the alkylation reaction if required.

2. A process according to claim 1 in which the isoparaffin is selected from the group constituted by isobutane and isopentane.

3. A process according to claim 1 or claim 2, in which the catalytic bed is selected from the group constituted by a fixed, expanded, ebullated or agitated bed, or an equivalent thereof.

4. A process according to any one of claims 1 to 3, in which extraction of the reaction effluent from the reaction zone and recycling to said reaction zone of an isoparaffin-rich fraction are carried out substantially simultaneously.

5. A process according to claim 4 or claim 5, characterised in that it is repeated periodically.

6. A process according to any one of claims 1 to 5, in which feed (i) is introduced at several points in the reaction zone, these points being located at different levels.

7. A process according to any one of claims 4 to 6, in which:
(a) the following compounds or elements are introduced into the reaction zone:
(i) a feed,
(ii) optionally, at least a portion of the reaction effluent,
(iii)an isoparaffin-rich effluent from the reaction effluent,
(b) after optionally stopping the alkylation reaction, extracting the reaction effluent and optionally recycling a portion of this reaction effluent designated (ii), to step (a) to the reaction zone,
(c) sending at least a portion of said reaction effluent to a separation or fractionation zone,
(d) recycling at least a portion of an isoparaffin-rich fraction extracted from said zone S and designated (iii) in step (a), to the reaction zone,
(e) recovering an alkylate as product from zone S.

8. A process according to claim 7, in which a n-paraffin is recovered from zone S as a purge.

9. A process according to claim 7 or claim 8, in which the catalytic bed is washed in the reaction zone using said isoparaffin-rich fraction extracted from said zone S and designated (iii) in step (a).

10. A process according to any one of claims 7 to 9, in which the recycle carried out at (a) of at least a portion of the reaction effluent (ii) is carried out at several levels in the reaction zone.

11. A process according to any one of claims 7 to 10, in which the recycle carried out at (a) of a portion of the isoparaffin-rich fraction (ii) is carried out at different levels of the catalytic bed.

12. A process according to any one of claims 7 to 11, in which in step (a), the elements (i), (ii) and optionally (iii) are mixed before being introduced into reaction zone R.

13. A process according to any one of claims 1 to 12 in which, before introduction of the feed into the reaction zone, the feed is dried over a molecular sieve and selectively hydrogenated to eliminate at least the major portion of the highly unsaturated compounds it may contain.

14. A process according to any one of claims 4 to 13, in which the ratio of the molar flow rates of the isoparaffin extracted from the separation or fractionation zone S and the alkylate extracted from the same zone is between 5:1 and 100:1.

15. A process according to claim 14, in which said ratio is between 10:1 and 50:1.

16. A process according to any one of claims 1 to 15, in which a catalytic bed rejuvenation treatment is carried out at a frequency corresponding to a catalyst age (expressed as the weight of alkylate produced per weight of catalyst) of between 0.01 and 50 g/g.

17. A process according to claim 4, in which the duration of said process is between 1 minute and 10 hours.

18. A process according to any one of claims 1 to 17, in which the alkylation reaction is carried out in at least two reaction zones disposed in series and in which the isoparaffin to be treated is introduced into the first reaction zone crossed by the feed, in which the olefin is introduced into each reaction zone and in which the isoparaffin extracted from zone S is recycled to the first reaction zone.

19. A process according to any one of claims 1 to 17, in which the alkylation reaction is carried out in at lesat two reaction zones disposed in parallel, each reaction zone being supplied with olefin to be treated, with isoparaffin to be treated and with recycled isoparaffin from zone S.

20. A process according to any one of claims 1 to 19 in which, after several catalytic rejuvenation treatments, at least a portion of said catalytic bed is extracted, said portion of catalytic bed being replaced by regenerated or fresh catalyst.

## Patentansprüche

1. Verfahren zur sogenannten Rejuvenation (Verjüngung) eines festen Katalysators zur Alkylierung wenigstens eines Isoparaffins durch wenigstens ein 3 bis 6 Kohlenstoffatome pro Molekül aufweisendes Olefin, wobei diese Rejuvenation in der Reaktionszone durch Zirkulation auf dem katalytischen Bett wenigstens eines Teils einer an Isoparaffin reichen Fraktion durchgeführt wird, die durch Fraktionierung oder Abtrennung des flüssigen aus der Reaktionszone stammenden Abstroms erhalten wurde, das Verfahren die Speisung einer ein katalytisches Bett umfassenden Reaktionszone R mit einer Charge (i) umfaßt, die wenigstens dieses Isoparaffin und dieses Olefin einschließt, die wenigstens zum Teil an der Basis der Reaktionszone eingeführt wird, dadurch gekennzeichnet, daß
- periodisch wenigstens die Speisung unterbrochen wird,
- man zum Abziehen des Reaktionsabstroms übergeht, wobei das katalytische Bett im wesentlichen in der Reaktionszone (R) verbleibt,
- man in einer Zone S den Reaktionsabtrom abtrennt oder fraktioniert, um wenigstens zum Teil eine an Isoparaffin reiche Fraktion zu sammeln, und wenigstens ein Teil dieser Fraktion in die Reaktionszone, um diesen Katalysator zum waschen, recycliert wird,
- man zum wiederanlaufen der Reaktion übergeht, indem man die Reaktionszone mit der Charge (i), ggf. unter Einstellung der zur Alkylierungsreaktion notwendigen Arbeitsbedingungen, speist.

2. Verfahren nach Anspruch 1, bei dem das Isoparaffin gewählt wird aus der durch Isobutan und Isopentan gebildeten Gruppe.

3. Verfahren nach einem der Ansprüche 1 und 2, bei dem das katalytische Bett ein Bett ist, das aus der Gruppe gewählt ist, welche durch die festen Betten, expandierten Betten, Siedebetten, Rührbetten oder Betten äquivalenter Art gebildet ist.

4. Verfahren nach einem der Ansprüche 1 bis 3, bei dem das Abziehen des Reaktionsabstroms aus der Reaktionszone und das Recyclieren in diese Reaktionszone einer an Isoparaffin reichen Fraktion im wesentlichen gleichzeitig vorgenommen werden.

5. Verfahren nach einem der Ansprüche 4 und 5, dadurch gekennzeichnet, daß es periodisch wiederholt wird.

6. Verfahren nach einem der Ansprüche 1 bis 5, bei dem diese Charge (i) an mehreren Punkten der Reaktionszone eingeführt wird, wobei diese Punkte sich auf unterschiedlichen Höhen befinden.

7. Verfahren nach einem der Ansprüche 4 bis 6, bei dem:
(a) man in diese Reaktionszone die folgenden Verbindungen oder Elemente einführt:
(i) eine Charge
(ii) ggf. wenigstens einen Teil des Reaktionsabstroms,
(iii) eine an Isoparaffin reiche aus dem Reaktionsabstrom stammende Fraktion,
(b) man nach eventueller Unterbrechung der Alkylierungsreaktion den Reaktionsabstrom abzieht und man ggf. zur Reaktionszone einen Teil dieses mit (ii) bezeichneten Reaktionsabstroms zur Stufe (a) recycliert,
(c) man wenigstens einen Teil dieses Reaktionsabstroms in eine Trenn- oder Fraktionierungszone S schickt,
(d) man gegen die Reaktionszone wenigstens einen Teil einer an Isoparaffin reichen, aus der Zone S abgezogenen und in Stufe (a) mit (iii) bezeichneten Fraktion recycliert,
(e) und man aus der Zone S ein Alkylat als Produkt gewinnt.

8. Verfahren nach Anspruch 7, bei dem man im übrigen als Reinigungsmittel ein n-Paraffin in der Zone S gewinnt.

9. Verfahren nach einem der Ansprüche 7 und 8, bei dem das katalytische Bett in der Reaktionszone vermittels dieser an Isoparaffin reichen, aus der Zone S abgezogenen und mit (iii) in Stufe (a) bezeichneten Fraktion gewaschen wird.

10. Verfahren nach einem der Ansprüche 7 bis 9, bei dem die Recyclierung, durchgeführt in (a), eines Teils des Reaktionsabstroms (ii) auf mehreren Niveaus der Reaktionszone realisiert wird.

11. Verfahren nach einem der Ansprüche 7 bis 10, bei dem die in (a) realisierte Recyclierung eines Teils der an Isoparaffin (iii) reichen Fraktion auf unterschiedlichen Niveaus dieses katalytischen Bettes durchgeführt wird.

12. Verfahren nach einem der Ansprüche 7 bis 11, bei dem in der Stufe (a) die Elemente (i), (ii) und ggf. (iii) vermischt werden, bevor sie in die Reaktionszone R eingeführt werden.

13. Verfahren nach einem der Ansprüche 1 bis 12, bei dem vorher, vor dem Einführen der Charge in die Reaktionszone, diese Charge auf einem Molekularsieb getrocknet und selektiv derart hydriert wird, daß der größere Teil wenigstens der stark ungesättigten Verbindungen, die diese enthalten kann, eliminiert wird.

14. Verfahren nach einem der Ansprüche 4 bis 13, bei dem das Verhältnis der Moldurchsätze an abgezogenem Isoparaffin aus der Trenn- oder Fraktionierungszone S und aus eben dieser Zone abgezogenem Alkylat zwischen 5:1 und 100:1 beträgt.

15. Verfahren nach Anspruch 14, bei dem das Verhältnis zwischen 10:1 und 50:1 beträgt.

16. Verfahren nach einem der Ansprüche 1 bis 15, bei dem eine "Rejuvenationsbehandlung" eines katalytischen Bettes mit einer Periodizität durchgeführt wird, die einem Alter des Katalysators (ausgedrückt in Gewicht erzeugten Alkylats pro Gewicht Katalysator) zwischen 0,01 und 50 g/g entspricht.

17. Verfahren nach Anspruch 4, dadurch gekennzeichnet, daß die Dauer dieses Verfahrens zwischen 1 Minute und 10 Stunden beträgt.

18. Verfahren nach einem der Ansprüche 1 bis 17, bei dem die Alkylierungsreaktion in wenigstens zwei in Reihe angeordneten Reaktionszonen durchgeführt wird und bei dem das zu behandelnde Isoparaffin in die erste von der Charge durchsetzte Reaktionszone, in der das Olefin in jede Reaktionszone eingeführt wird, eingeführt wird und in der das aus der Zone S abgezogene Isoparaffin zur ersten Reaktionszone recycliert wird.

19. Verfahren nach einem der Ansprüche 1 bis 17, bei dem die Alkylierungsreaktion in wenigstens zwei parallel angeordneten Reaktionszonen durchgeführt wird, wobei jede Reaktionszone mit zu behandelndem Olefin, mit zu behandelndem Isoparaffin sowie mit aus der Zone S stammendem Recycle-Isoparaffin gespeist wird.

20. Verfahren nach einem der Ansprüche 1 bis 19, bei dem bei Ende der Behandlungen der katalytischen Rejuvenation man wenigstens einen Teil des katalytischen Bettes abzieht, wobei dieser Teil des katalytischen Bettes durch den regenerierten Katalysator oder durch frischen Katalysator ersetzt wird.
